# EUROPEAN PATENT APPLICATION

(11) **EP 2 574 282 A1**
(43) Date of publication of application: **03.04.2013**
(21) Application number: 12185958.1
(22) Date of filing: 25.09.2012
(51) Int. Cl.: A61B 8/00, A61B 19/00

(54) **Ultrasound diagnostic apparatus and method thereof**

(30) Priority: 27.09.2011 JP 2011211323
(71) Applicant: GE Medical Systems Global Technology Company LLC, Waukesha, Wisconsin 53188-1696 (US)
(72) Inventor: Hashimoto, Hiroshi, Hino-shi Tokyo 191-8503 (JP); Sato, Naoto, Hino-shi Tokyo 191-8503 (JP)
(74) Representative: Bedford, Grant Richard

(57) **Abstract**

An ultrasound diagnostic apparatus 1 includes: an ultrasound probe 2 which performs transmission/reception of ultrasound on a subject; and an indicator display control unit 56 adpted to display indicators each indicative of a distance between at least a part of an outline of a transmission/reception region of real-time ultrasound set in advance of the subject and a corresponding position stored in advance of the outline.

## Description

The present invention relates generally to an ultrasound diagnostic apparatus in which an ultrasound image and a reference medical image are both displayed, and a method thereof.

In an ultrasound diagnostic apparatus, an ultrasound image is generated based on echo signals obtained by performing the transmission of ultrasound. A real-time ultrasound image can also be displayed. As such an ultrasound diagnostic apparatus, there has been disclosed in, for example, a patent document 1, an ultrasound diagnostic apparatus which displays a real-time ultrasound image and a reference medical image such as an X-ray CT (Computed Tomography) image, an MRI (Magnetic Resonance Imaging) image, or the like both identical in section at a subject. In the ultrasound diagnostic apparatus, a region corresponding to the position of an ultrasound image is specified in volume data acquired by an X-ray CT apparatus or an MRI system, based on the position of an ultrasound probe detected by a position sensor. The reference medical image is displayed with respect to the corresponding region. Thus, even if the ultrasound probe is moved, the reference medical image is also allowed to follow it, so that an image identical in region to the ultrasound image is always displayed. It is thus possible to easily contrast the ultrasound image and the reference medical image with each other.

[Patent Document 1] International Patent Publication No. W02004-098414, Pamphlet

On the other hand, when, for example, a biopsy needle is inserted in a biological tissue to perform curing or take a tissue, it is necessary to avoid bones, a large vessel, etc. Thus, before the biopsy needle is inserted therein, whether the biopsy needle should be inserted from any part and angle is specified while looking at an ultrasound image, and a plan for its insertion is developed. Then, the biopsy needle is inserted while displaying an ultrasound image identical in region to a transmission/reception region of ultrasound specified at the insertion plan.

Here, there is a case in which the date of making the insertion plan and the date of performing the biopsy are different. A person different from a person for the insertion plan may carry out biopsy. In such a case, there is a need to, at the insertion of the biopsy needle, search the transmission/reception region of the ultrasound specified at the insertion plan and display the ultrasound image in the same region. There has thus been a demand for an ultrasound diagnostic apparatus capable of causing a transmission/reception region of real-time ultrasound to easily coincide with a transmission/reception region of ultrasound specified in advance.

The invention according to one aspect relates to an ultrasound diagnostic apparatus equipped with an ultrasound probe which performs transmission/reception of ultrasound on a subject, and an indicator display control unit which causes to be displayed indicators each indicative of a distance between at least part set in advance, of an outline of a transmission/reception region of real-time ultrasound at the subject and a corresponding position stored in advance at the outline.

According to the invention of the above aspect, the indicators each indicative of the distance between at least part set in advance, of the outline of the transmission/reception region of real-time ultrasound at the subject and the corresponding position stored in advance at the outline are displayed. It is therefore possible to cause the transmission/reception region of the real-time ultrasound and the transmission/reception region of ultrasound specified in advance to easily coincide with each other. Since the preset part of the outline of the transmission/reception region is stored, it is possible to save the time and effort required for an operator to specify the stored part.

Various objects and advantages of the present invention will be apparent from the following description of the preferred embodiments of the invention as illustrated in the accompanying drawings, in which:
FIG. 1 is a block diagram showing one example of a schematic configuration of an ultrasound diagnostic apparatus according to a first embodiment of the invention.
FIG. 2 is a block diagram illustrating a configuration of a display controller in the ultrasound diagnostic apparatus according to the first embodiment.
FIG. 3 is a flowchart for describing determination of an insertion position of a biopsy needle in the ultrasound diagnostic apparatus according to the first embodiment.
FIG. 4 is a diagram depicting one example of a display unit on which an ultrasound image and a reference medical image are displayed.
FIG. 5 is a diagram showing one example of the display unit on which an ultrasound image and a reference medical image about the same section of a subject are displayed.
FIG. 6 is a diagram for describing four points at an outline of a transmission/reception region of ultrasound.
FIG. 7 is a diagram for describing the storage of information of positions corresponding to the corners of an outline of a transmission/reception region of ultrasound in volume data of a reference medical image.
FIG. 8 is a flowchart for describing the insertion of the biopsy needle.
FIG. 9 is a diagram showing the display unit which displays indicators on a reference medical image.
FIG. 10 is a diagram for describing distances indicated by indicators.
FIG. 11 is a diagram showing a positional relationship between a plane corresponding to a transmission/reception plane of a real-time ultrasound image in volume data and a region surrounded by an outline including each corner stored in a memory.
FIG. 12 is a diagram illustrating the display unit which displays another example illustrative of indicators on a reference medical image.
FIG. 13 is a diagram depicting the display unit which displays a further example illustrative of indicators on a reference medical image.
FIG. 14 is a diagram showing one example of a display unit which displays indicators on an ultrasound image in a first modification of the first embodiment.
FIG. 15 is a diagram illustrating another example of the display unit which displays indicators on an ultrasound image in the first modification of the first embodiment.
FIG. 16 is a diagram depicting one example of a display unit on which an enlarged ultrasound image and a reference medical image are displayed in a second modification of the first embodiment.
FIG. 17 is a diagram for describing a relationship between an ultrasound transmission/reception region and a display region of an ultrasound image.
FIG. 18 is a diagram showing one example of a display unit on which indicators are displayed in the second modification of the first embodiment.
FIG. 19 is a diagram illustrating another example of the display unit on which indicators are displayed in the second modification of the first embodiment.
FIG. 20 is a diagram showing a further example of the display unit on which indicators are displayed in the second modification of the first embodiment.
FIG. 21 is a diagram illustrating a still further example of the display unit on which indicators are displayed in the second modification of the first embodiment.
FIG. 22 is a flowchart for describing determination of an insertion position of a biopsy needle in a second embodiment.
FIG. 23 is a diagram for describing the storage of information about each position corresponding to the entirety of an outline of a transmission/reception region of ultrasound in volume data of a reference medical image.
FIG. 24 is a diagram showing one example of a display unit on which an indicator is displayed in the second embodiment.
FIG. 25 is a diagram illustrating another example of the display unit on which an indicator is displayed in the second embodiment.
FIG. 26 is a diagram showing a positional relationship between a plane corresponding to a transmission/reception plane of a real-time ultrasound image in volume data and a plane surrounded by an outline stored in a memory.
FIG. 27 is a diagram illustrating a further example of the display unit on which an indicator is displayed in the second embodiment.
FIG. 28 is a diagram depicting a still further example of the display unit on which an indicator is displayed in the second embodiment.
FIG. 29 is a diagram showing one example of a display unit which displays an indicator on an ultrasound image in a first modification of the second embodiment.
FIG. 30 is a diagram illustrating another example of the display unit which displays an indicator on an ultrasound image in the first modification of the second embodiment.
FIG. 31 is a diagram depicting one example of a display unit on which an indicator is displayed in a second modification of the second embodiment.
FIG. 32 is a diagram showing another example of the display unit on which an indicator is displayed in the second modification of the second embodiment.
FIG. 33 is a diagram showing one example of a display unit which displays an indicator on an ultrasound image in the second modification of the second embodiment.
FIG. 34 is a diagram illustrating another example of the display unit which displays an indicator on an ultrasound image in the second modification of the second embodiment.
FIG. 35 is a flowchart for describing determination of an insertion position of a biopsy needle in an ultrasound diagnostic apparatus according to a third embodiment.
FIG. 36 is a diagram showing a display unit on which an ultrasound image is displayed in the third embodiment.
FIG. 37 is a flowchart for describing the insertion of the biopsy needle in the third embodiment.
FIG. 38 is a diagram showing the display unit on which indicators are displayed in the third embodiment.
FIG. 39 is a diagram illustrating another example of the display unit on which indicators are displayed in the third embodiment.
FIG. 40 is a flowchart for describing determination of an insertion position of a biopsy needle in a first modification of the third embodiment.
FIG. 41 is a diagram showing a display unit on which an indicator is displayed in the first modification of the third embodiment.
FIG. 42 is a diagram illustrating another example of the display unit on which an indicator is displayed in the first embodiment of the third embodiment.
FIG. 43 is a flowchart for describing determination of an insertion position of a biopsy needle in a second modification of the third embodiment.
FIG. 44 is a flowchart for describing determination of an insertion position of a biopsy needle in a fourth modification of the third embodiment.
FIG. 45 is a diagram showing another example of the display unit on which indicators are displayed in the second modification of the first embodiment.
FIG. 46 is a diagram illustrating a further example of the display unit on which an indicator is displayed in the second modification of the second embodiment.

Various embodiments of the invention will hereinafter be described.

### First embodiment

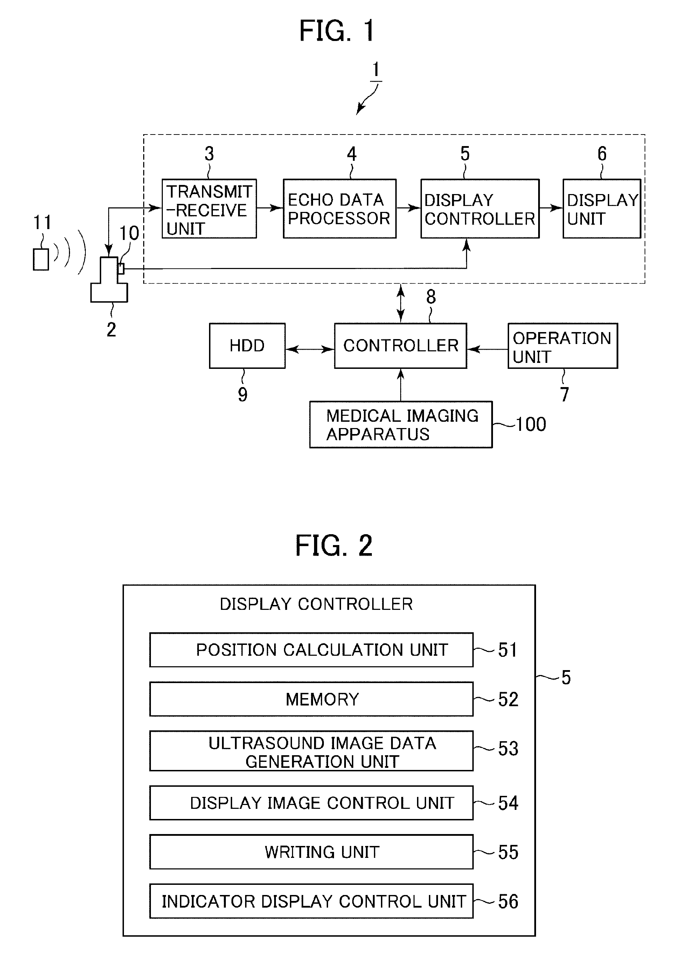

A first embodiment will first be described based on FIGS. 1 through 11. An ultrasound diagnostic apparatus 1 shown in FIG. 1 is equipped with an ultrasound probe 2, a transmit-receive unit 3, an echo data processor 4, a display controller 5, a display unit 6, an operation unit 7, a controller 8 and an HDD (Hard Disk Drive) 9.

The ultrasound probe 2 is comprised of a plurality of ultrasound transducers (not shown) arranged in array form. The ultrasound probe 2 transmits ultrasound to a subject through the ultrasound transducers and receives its echo signals.

The ultrasound probe 2 is provided with a magnetic sensor 10 comprised of, for example, a Hall element. The magnetic sensor 10 detects magnetic field generated from a magnetic generation unit 11 comprised of, for example, a magnetic generation coil. A signal detected by the magnetic sensor 10 is inputted to the display controller 5. The signal detected by the magnetic sensor 10 may be inputted to the display controller 5 through an unillustrated cable or may be inputted to the display controller 5 by radio. The magnetic generation unit 11 and the magnetic sensor 10 are used to detect the position and tilt of the ultrasound probe 2 as will be described later.

The transmit-receive unit 3 supplies an electric signal for transmitting ultrasound from the ultrasound probe 2 under a predetermined scan condition to the ultrasound probe 2, based on a control signal from the controller 8. The transmit-receive unit 3 performs signal processing such as A/D conversion, phasing-adding processing, etc. on each echo signal received by the ultrasound probe 2 and outputs echo data after the signal processing to the echo data processor 4.

The echo data processor 4 performs processing for generating an ultrasound image on the echo data outputted from the transmit-receive unit 3. For example, the echo data processor 4 performs B-mode processing such as logarithmic compression processing, envelop detection processing or the like to thereby generate B-mode data.

The display controller 5 has a position calculation unit 51, a memory 52, an ultrasound image data generation unit 53, a display image control unit 54, a writing unit 55, and an indicator display control unit 56 as shown in FIG. 2. The position calculation unit 51 calculates information (hereinafter called "probe position information") about the position and tilt of the ultrasound probe 2 in a coordinate system of a three-dimensional space with the magnetic generation unit 11 as an origin point, based on the magnetic detection signal from the magnetic sensor 10. Further, the position calculation unit 51 calculates position information about each echo signal in the coordinate system of the three-dimensional space, based on the probe position information. The coordinate system of the three-dimensional space with the magnetic generation unit 11 as the origin point is assumed to be called a coordinate system of an ultrasound image UG.

The memory 52 is comprised of a semiconductor memory or the like such as a RAM (Random Access Memory), a ROM (Read Only Memory), or the like. For example, data and the like outputted from the echo data processor 4 and prior to being converted to ultrasound image data at the ultrasound image data generation unit 53 as will be described later are stored in the memory 52. The data prior to being converted to the ultrasound image data is assumed to be called raw data. The raw data may be stored in the HDD 9.

Position information about at least part of a transmission/reception region of ultrasound at the subject is stored in the memory 52. The position information is calculated by the position calculation unit 51. In the present embodiment, position information about four points at the outline of the ultrasound transmission/reception region is stored. The details thereof will be described later. The position information may be stored in another storage unit such as the HDD 9 or the like.

Volume data of a medical image (to be described later) acquired in advance by a medical imaging apparatus 100 may be stored in the memory 52 through the controller 8.

The ultrasound image data generation unit 53 scan-converts data inputted from the echo data processor 4 by means of a scan converter to generate ultrasound image data.

The display image control unit 54 causes the display unit 6 to display a real-time ultrasound image UG and causes a reference medical image MG identical in section at the subject to the transmission/reception region of ultrasound calculated by the position calculation unit 51 to be displayed.

The reference medical image MG is a medical image other than the real-time ultrasound image UG. Described specifically, the reference medical image MG may be a medical image acquired in advance by the medical imaging apparatus 100 other than the ultrasound diagnostic apparatus 1, i.e., an X-ray CT image or an MRI image acquired in advance by, for example, an X-ray CT apparatus or an MRI system or the like. The reference medical image MG may be an ultrasound image acquired in advance. The ultrasound image may be one acquired at the ultrasound diagnostic apparatus 1 or may be one acquired at another ultrasound diagnostic apparatus not shown in the drawing.

The writing unit 55 writes position information about at least part of an ultrasound transmission/reception region at the subject into the memory 52. The details thereof will be described later.

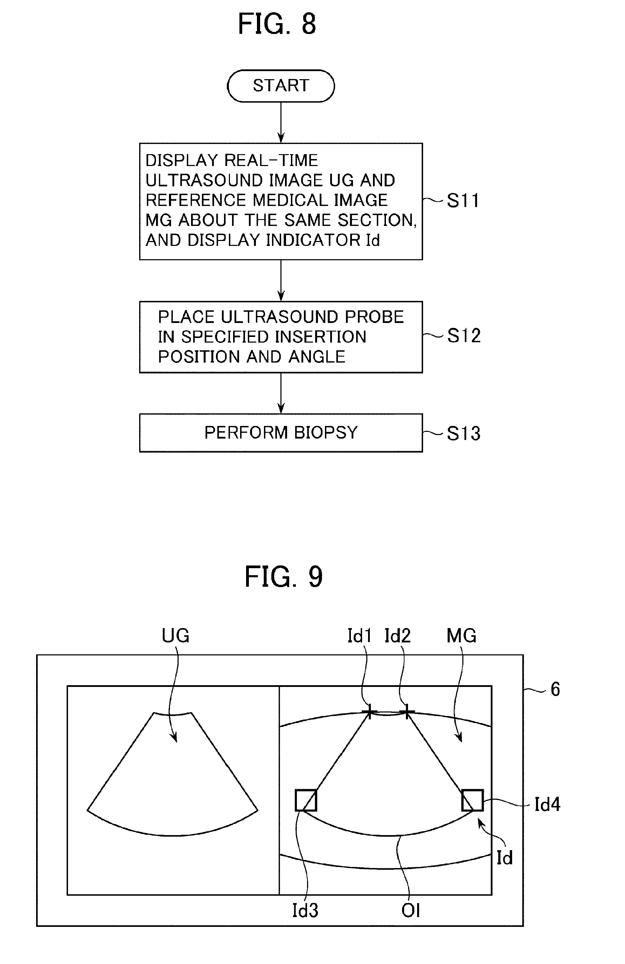

The indicator display control unit 56 causes an indicator Id (refer to FIG. 9) indicative of the distance between at least part set in advance, of the outline of the transmission/reception region of the real-time ultrasound about the subject, in which volume data of the reference medical image MG has been stored, and its corresponding position stored in the memory 52, of the outline to be displayed on the reference medical image MG (indicator display control function).

The display unit 6 is an LCD (Liquid Crystal Display), a CRT (Cathode Ray Tube) or the like. The operation unit 7 includes a keyboard and a pointing device (not shown) or the like for inputting instructions and information by an operator.

The controller 8 has a CPU (Central Processing Unit) although not shown in particular. The controller 8 reads a control program stored in the HDD 9 to execute functions at the respective parts of the ultrasound diagnostic apparatus 1 starting with the indicator display control function.

The data of the reference medical image MG acquired in advance with respect to the same subject as a target for transmission/reception of ultrasound is stored in the HDD 9 in addition to the control program. The data of the reference medical image MG is of volume data about a three-dimensional region at a subject. The data of the reference medical image MG may be stored in the memory 52. The data of the reference medical image MG is stored in the HDD 9 together with the position information in the coordinate system of the reference medical image MG. Incidentally, when the reference medical image MG is of an ultrasound image, position information acquired by detecting the position of the ultrasound probe 2 is stored.

The operation of the ultrasound diagnostic apparatus 1 according to the present embodiment will now be explained. A description will be made of, as an example, a case where after the position of insertion of the biopsy needle at the subject has been determined, the biopsy needle is inserted into the position using the ultrasound diagnostic apparatus 1.

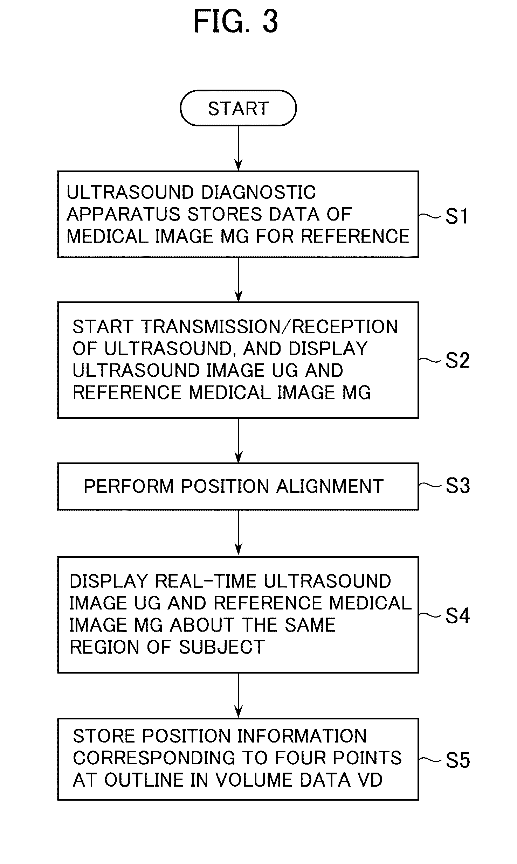

The determination of the position of insertion of the biopsy needle will first be described. The operator determines the position to insert the biopsy needle while looking at a real-time ultrasound image UG and a reference medical image MG of the same section as the ultrasound image UG. This will concretely be explained based on the flowchart shown in FIG. 3. At Step S1 of FIG. 3, data (volume data) of the reference medical image acquired in advance by the medical imaging apparatus 100 is first stored in the HDD 9 or the memory 52.

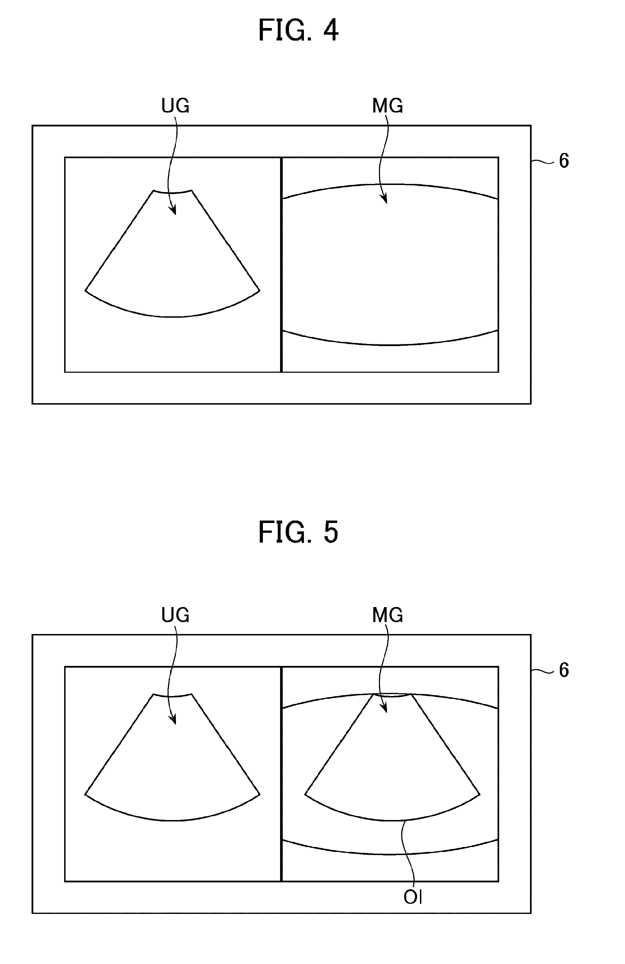

Next, at Step S2, the operator brings the ultrasound probe 2 into contact with a body surface of a subject and starts transmission/reception of ultrasound. Then, the display controller 5 causes the display unit 6 to display an ultrasound image UG generated based on each echo signal. The ultrasound image UG is of, for example, a B-mode image. Also the display controller 5 causes the display unit 6 to display a reference medical image MG, based on the volume data stored in the HDD 9 or the memory 52. As shown in FIG. 4, the display controller 5 allows the display unit 6 to display the ultrasound image UG and the reference medical image MG side by side.

Next, at Step S3, the process of aligning a coordinate system of the ultrasound image UG with a coordinate system of the reference medical image MG is performed. Described concretely, the operator moves the section of either of the ultrasound image UG and the reference medical image MG both displayed on the display unit 6 or the sections of both images displayed thereon while comparing the ultrasound image UG and the reference medical image MG and displays the ultrasound image UG and the reference medical image MG identical in section on the display unit 6. The movement of the section of the ultrasound image UG is performed by changing the position of the ultrasound probe 2. The movement of the section of the reference medical image MG is performed by inputting an instruction for operating the operation unit 7 to thereby change the section.

Whether they are identical in section is determined by, for example, reference to a characteristic region by the operator, or the like. When the ultrasound image UG and the reference medical image MG identical in section are displayed, the operator designates an arbitrary point of the ultrasound image UG using a track ball or the like of the operation unit 7. The operator designates a point considered to be the same position as the point designated at the ultrasound image UG even with respect to the reference medical image MG. The operator performs such point designation on a plurality of points.

Here, the data of the reference medical image MG has position information. Thus, when the point considered to be the same position is designated with respect to the ultrasound image UG and the reference medical image MG as described above, the position of correspondence between the coordinate system of the ultrasound image UG and the coordinate system of the reference medical image MG is specified. Coordinate transformation between the coordinate system of the ultrasound image UG and the coordinate system of the reference medical image MG is made possible by specifying the point of correspondence between the coordinate system of the ultrasound image UG and the coordinate system of the reference medical image MG in plural form. The alignment processing is completed as described above.

When the alignment processing at Step S3 is completed, the display image control unit 54 causes the reference medical image MG about the section corresponding to the position of each echo signal calculated by the position calculation unit 51 to be displayed together with the real-time ultrasound image UG. Thus, the ultrasound image UG and the reference medical image MG identical in section at the subject are displayed.

Described concretely, the display image control unit 54 coordinate-transforms the position information about the echo signal calculated by the position calculation unit 51 into position information of the coordinate system of the reference medical image MG and thereby specifies a region corresponding to the position of the echo signal in the volume data VD of the reference medical image MG. Next, the display image control unit 54 causes a reference medical image MG based on data of the section including this corresponding region to be displayed together with a real-time ultrasound image UG as shown in FIG. 5. In FIG. 5, the region surrounded by an outline or profile line Ol displayed on the reference medical image MG is a region corresponding to the ultrasound image UG at the reference medical image MG. An image lying within the outline Ol at the reference medical image MG, and the ultrasound image UG are images in the same region at the subject. The outline Ol is specified by coordinate-transforming the position of echo data in the coordinate system of the ultrasound image UG, which has been calculated by the position calculation unit 51, into the coordinate system of the reference medical image MG.

In the present embodiment, the reference medical image MG is displayed with respect to a range wider than the outline Ol. Thus, a reference medical image MG having a range wider than the corresponding region of the ultrasound image UG is displayed.

Next, at Step S5, the operator moves the ultrasound probe 2 while looking at the real-time ultrasound image UG and the reference medical image MG about the same section at the subject and searches for the position to insert the biopsy needle and its insertion angle while changing an angle relative to the body surface. When the operator finds a suitable position and angle, the operator performs, through the operation unit 7, the input of storing position information of four points at the outline of an ultrasound transmission/reception region.

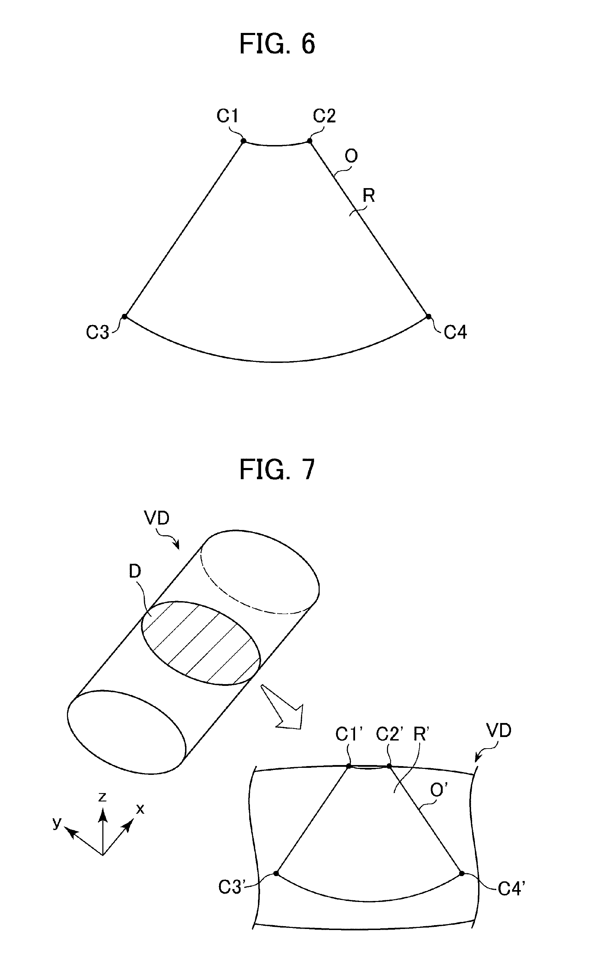

The four points of the outline of the ultrasound transmission/reception region are four corners C1, C2, C3 and C4 at an outline O of an ultrasound transmission/reception region R as shown in FIG. 6. When the input of storing the position information is done at the operation unit 7, the writing unit 55 stores information about the positions corresponding to the corners C1 through C4 in the volume data of the reference medical image into the memory 52. If described in more detail, a section D at the volume data VD of the reference medical image is a section (the same section at the subject) corresponding to the section of the ultrasound transmission/reception region R as shown in FIG. 7. The writing unit 55 writes position information about corners C1', C2', C3' and C4' at an outline O' of a region R' corresponding to the transmission/reception region R at the section D into the memory 52.

Which part (the corners C1' through C4' in the present embodiment) of the outline O' should be stored in the memory 52 has been set in advance.

Incidentally, points corresponding to at least three points at the outline O of the ultrasound transmission/reception region R may be stored in the memory 52.

The insertion of the biopsy needle in the position specified at Step S5 will next be explained based on the flowchart shown in FIG. 8. At Step S11, a real-time ultrasound image UG and a reference medical image MG identical in section to the ultrasound image UG are first displayed on the display unit 6. Described concretely, the operator performs transmission/reception of ultrasound on the subject at which the position of insertion of the biopsy needle has been determined at Steps S1 through S5, by means of the ultrasound probe 2. A real-time ultrasound image UG and a reference medical image MG identical in section to the ultrasound image UG are displayed on the display unit 6. When the alignment processing at the above Step S3 is however required again, the operator performs the alignment processing again.

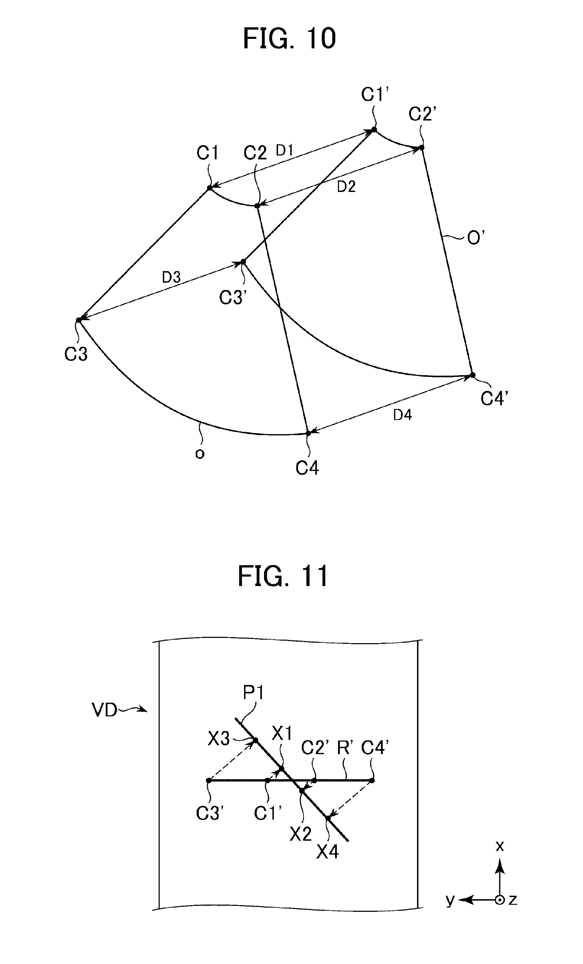

At Step S11, the indicator display control unit 56 causes indicators Id1, Id2, Id3 and Id4 to be displayed at the corners of the outline Ol in the reference medical image MG as shown in FIG. 9. The indicators Id1 through Id4 indicate the distances between the corners C1, C2, C3 and C4 of the transmission/reception region R of the real-time ultrasound image UG at the subject and the corners C1', C2', C3' and C4' respectively. They will be explained in detail based on FIG. 10. In FIG. 10, an outline o is a part corresponding to the outline O of the transmission/reception R of the real-time ultrasound image UG in the volume data VD (not shown in FIG. 10). The corners C1', C2', C3' and C4' are respectively points corresponding to the corners C1, C2, C3 and C4 at the outline O. The indicator Id1 indicates a distance D1 between the corner C1 and the corner C1', the indicator Id2 indicates a distance D2 between the corner C2 and the corner C2', the indicator Id3 indicates a distance D3 between the corner C3 and the corner C3', and the indicator Id4 indicates a distance D4 between the corner C4 and the corner C4'. The indicator display control unit 56 calculates the distances D1 through D4 and causes the indicators Id1 through Id4 to be displayed.

The indicators Id1 through Id4 are quadrangles having areas corresponding to the distances D1 through D4. When the distances D1 through D4 are zero, the indicators Id1 through Id4 become graphics of "+".

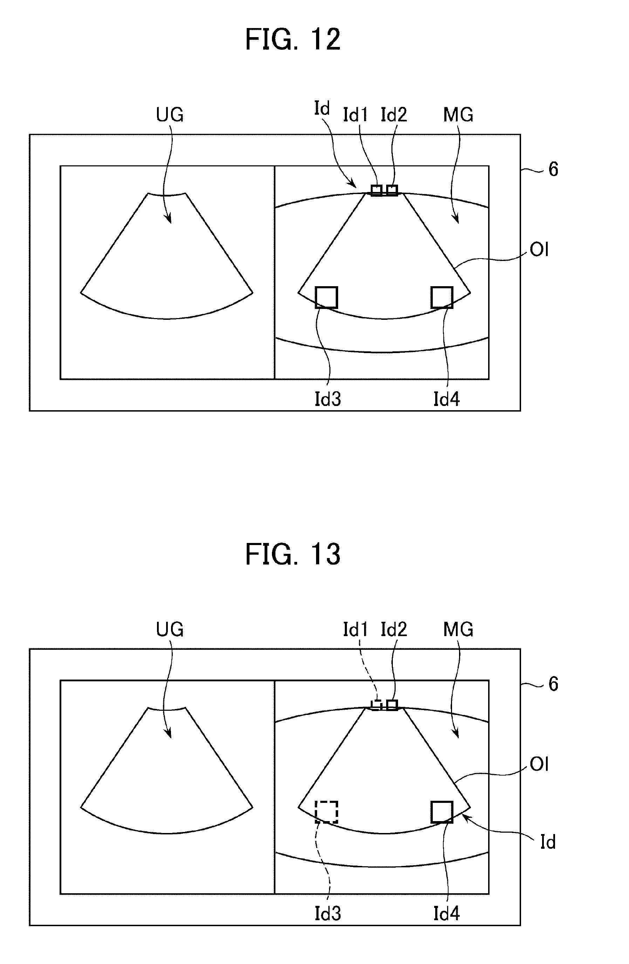

However, the indicator display control unit 56 may display the indicators Id1 through Id4 at their corresponding positions where the corners C1', C2', C3' and C4' are projected on the reference medical image MG other than the outline Ol, i.e., positions where the corners C1', C2', C3' and C4' are projected on a plane P1 corresponding to the transmission/reception plane of real-time ultrasound in the volume data VD. In this case, when the plane P1 corresponding to the transmission/reception plane of the real-time ultrasound and the region R' intersects each other in the volume data (FIG. 11 is a diagram of the volume data VD shown in FIG. 7 as viewed from a z-axis direction) as shown in FIG. 11, for example, the indicators Id1 through Id4 are respectively displayed on positions X1, X2, X3 and X4 (refer to FIG. 11) where the corners C1', C2', C3' and C4' are projected onto the plane P1 other than the outline Ol at the reference medical image MG as shown in FIG. 12. In this case, the indicator display control unit 56 may change the form of the indicator Id as shown in FIG. 13 according to on which side the corners C1 through C4 of the transmission/reception region R of the real-time ultrasound are located with respect to the corners C1' through C4'. In FIG. 13, the indicators Id are displayed in solid and broken lines. It is thus possible to easily grasp in which positional relation the transmission/reception plane of the real-time ultrasound image UG is placed with respect to the section including the corners C1' through C4' stored in the memory 52.

Next, at Step S12, the operator places the ultrasound probe 2 in the insertion position and angle both specified at Step S5. The operator adjusts the position and angle of the ultrasound probe 2 and thereby searches for the insertion position and angle specified at Step S5. At this time, the operator adjusts the position and angle of the ultrasound probe 2 in such a manner that the indicators Id1 through Id4 reach graphics of "+". When the ultrasound probe 2 is placed in the insertion position and angle both specified at Step S5, the operator inserts the biopsy needle at Step S13.

According to the ultrasound diagnostic apparatus 1 of the present embodiment as described above, the operator is capable of causing the transmission/reception region of the real-time ultrasound to easily coincide with an ultrasound transmission/reception region specified in advance by referring to the indicator Id. Thus, when the operator inserts the biopsy needle while looking at the ultrasound image UG, the operator is able to easily specify the insertion position and angle of the biopsy needle both specified in advance.

Since the preset corners C1' through C4' of the outline O' of the region R' are stored in the memory 52, it is possible to save time and effort designated by the operator.

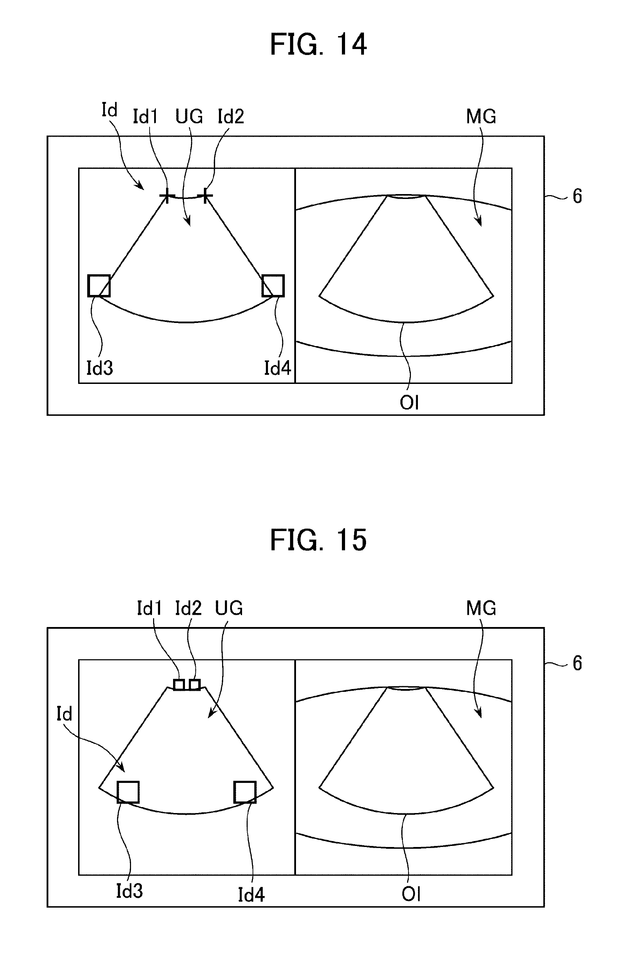

Modifications of the first embodiment will next be explained. A first modification will first be described. In the first modification, the indicator display control unit 56 may cause the indicators Id1 through Id4 to be displayed on a real-time ultrasound image UG as shown in FIG. 14. In this case, the indicators Id1 through Id4 are respectively displayed at the four corners of the ultrasound image UG, i.e., the positions corresponding to the corners C1 through C4.

Even in the first medication, as shown in FIG. 15, the indicator display control unit 56 may cause the indicators Id1 through Id4 to be displayed respectively at positions where the corners C1' through C4' are projected onto the real-time ultrasound image UG, i.e., positions where the corners C1' through C4' are projected onto the transmission/reception plane of the real-time ultrasound.

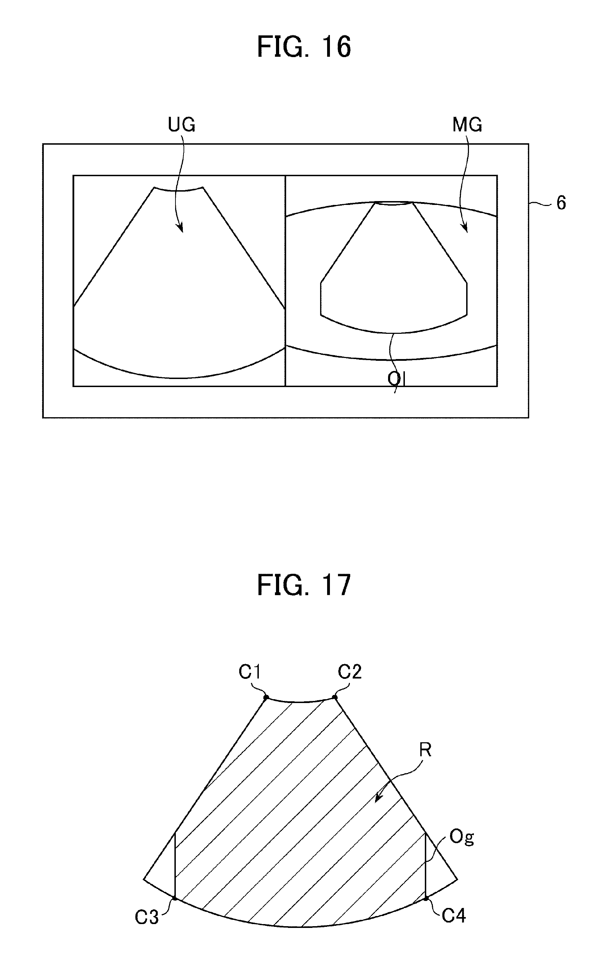

A second modification will next be explained. In the above description, the ultrasound image UG is displayed over the entirety of the transmission/reception region R of the ultrasound. The ultrasound transmission/reception region R and the display region of the ultrasound image UG coincide with each other. When the ultrasound image UG is however displayed in an enlarged form as shown in FIG. 16, for example, there is a case in which parts of the ultrasound transmission/reception region R shown in FIG. 17 are not displayed (the display region of the ultrasound image UG is shown in oblique lines in FIG. 17). Thus, when the transmission/reception region R of the ultrasound and the display region of the ultrasound image UG do not coincide with each other, points (corners C1' through C4' (not shown herein)) corresponding to corners C1, C2, C3 and C4 at the outline of the display region of the ultrasound image UG in volume data of a reference medical image are stored at Step S5. Even in this case, the positions (the corners C1 through C4) to be stored have been set in advance. The corners of the display region of the ultrasound image UG are at least parts of the outline of the ultrasound transmission/reception region.

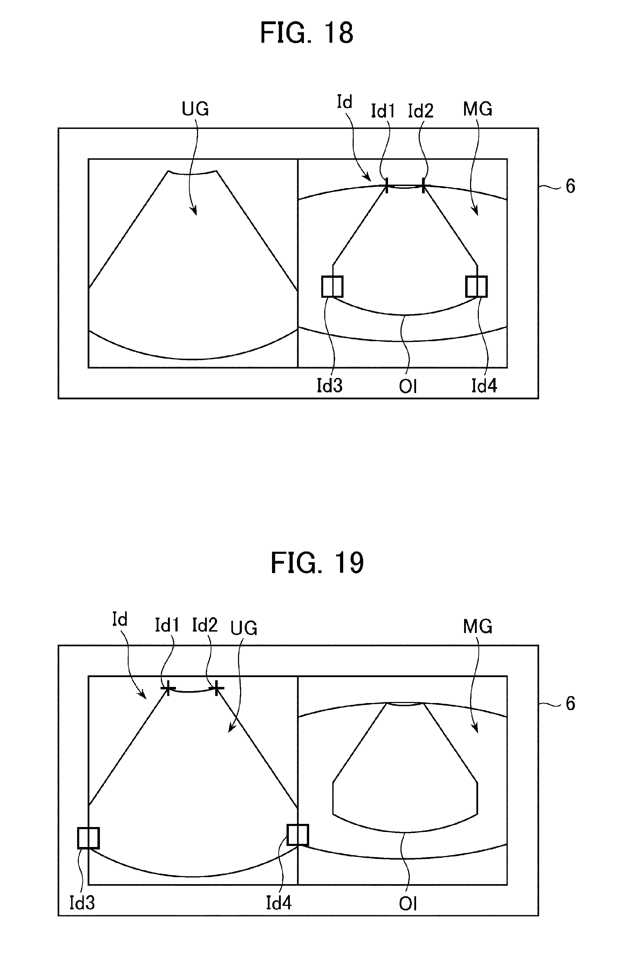

In the second medication, the indicator Id is displayed at points on the outline Ol, corresponding to the corners C1 through C4 of the display region as shown in FIG. 18.

In the second modification, the indicator display control unit 56 may cause the indicator Id to be displayed on a real-time ultrasound image UG as shown in FIG. 19. In this case, the indicators Id1 through Id4 are respectively displayed at points corresponding to the corners C1 through C4 of the ultrasound image UG.

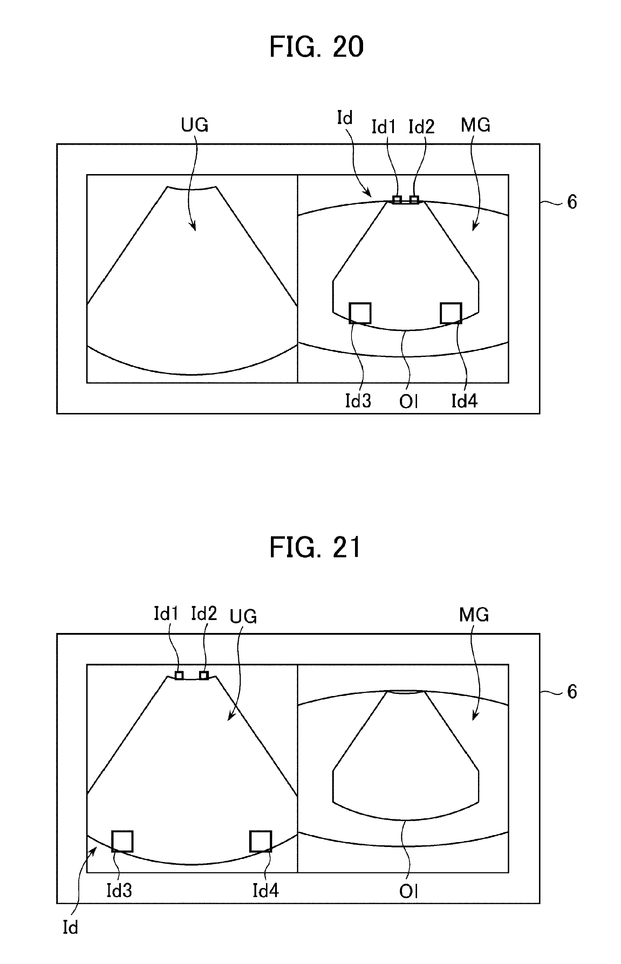

In the second modification, as shown in FIG. 20, the indicator display control unit 56 may cause the indicators Id1 through Id4 to be displayed respectively at positions where the corners C1' through C4' are projected onto the reference medical image MG. Further, as shown in FIG. 21, the indicator display control unit 56 may cause the indicators Id1 through Id4 to be displayed respectively at positions where the corners C1' through C4' are projected onto the real-time ultrasound image UG.

### Second embodiment

A second embodiment will next be explained. The same matters as those in the first embodiment will however not be described.

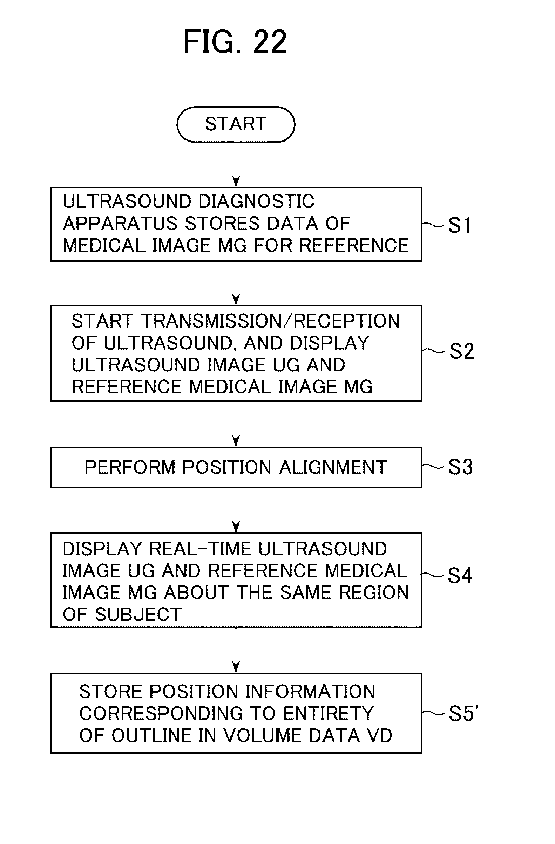

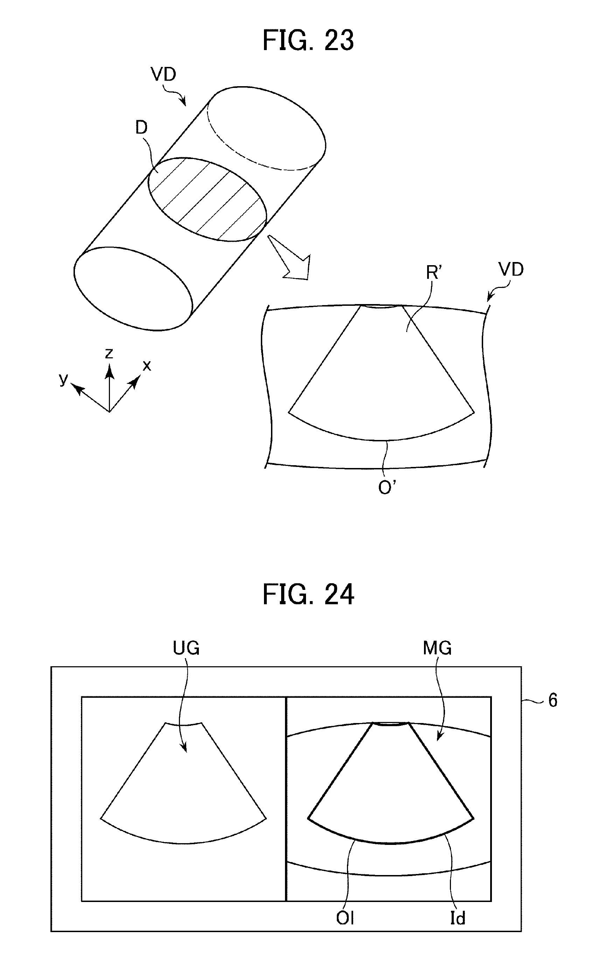

The operation of the present embodiment will be explained based on the flowchart of FIG. 22. Steps S1 through S4 are the same as those in the flowchart shown in FIG. 3. At Step S5', as shown in FIG. 23, position information about an outline O' corresponding to the entirety of the outline O of the ultrasound transmission/reception region R in the volume data VD of the reference medical image is stored in the memory 52.

The insertion of the biopsy needle into the position specified at Step S5' is performed in accordance with Step S11 through S13 shown in FIG. 8 even in the present embodiment. The present embodiment is however different from the first embodiment in the display form of the indicator Id. In the present embodiment, as shown in FIG. 24, the indicator display control unit 56 causes the outline Ol in the reference medical image MG to be displayed in colors corresponding to the distance between the outline O' in the volume data VD and the outline o (refer to FIG. 10) corresponding to the outline O of the transmission/reception region R of the real-time ultrasound image UG. That is, the indicator display control unit 56 calculates the distance between each point (point corresponding to a pixel) at the outline O' stored in the memory 52 and each point (point corresponding to a pixel) at the outline o corresponding to the individual points in the volume data VD and causes parts corresponding to the individual points at the outline Ol to be displayed in colors corresponding to the distances. Accordingly, the indicator Id corresponds to the color of the outline Ol.

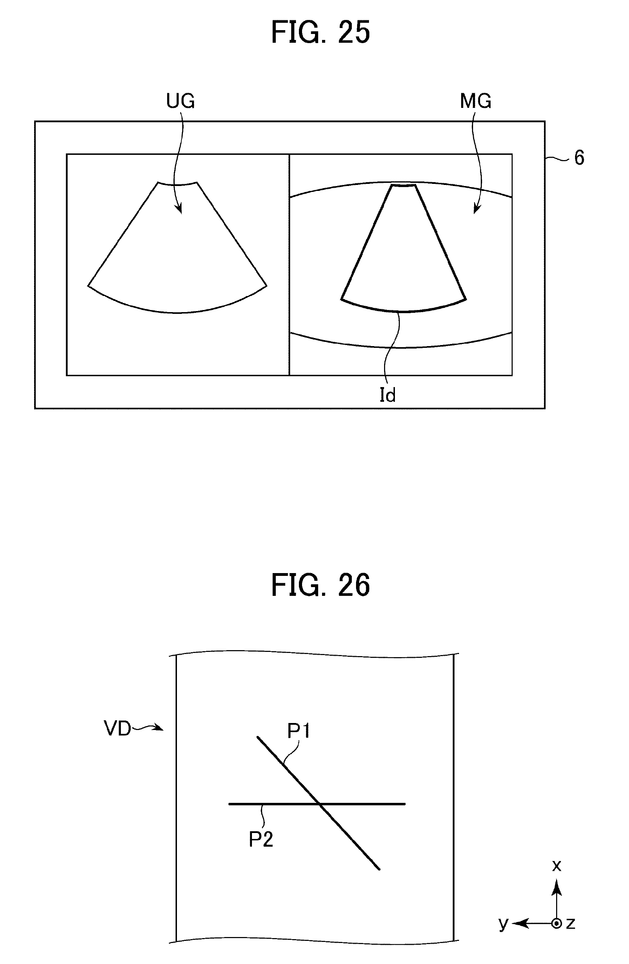

As shown in FIG. 25, the indicator display control unit 56 may cause the indicator Id to be displayed at a position where the outline O' is projected onto the reference medical image MG. Incidentally, the outline Ol is not displayed in FIG. 25.

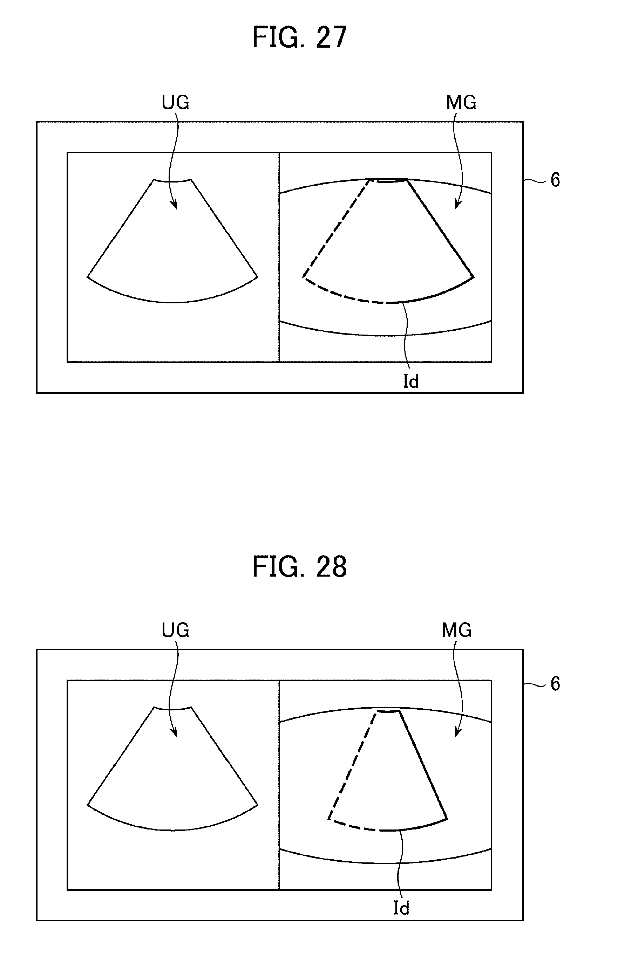

Here, the indicator display control unit 56 may change the display form of the indicator Id according to on which side a transmission/reception plane of a real-time ultrasound image UG is located with respect to the plane surrounded by the outline O' stored in the memory 52. When a plane P1 corresponding to a transmission/reception plane of a real-time ultrasound image UG intersects with a plane P2 surrounded by the outline O' in volume data VD as shown in FIG. 26, for example, one of two sections in the indicator Id may be displayed in solid line and the other thereof may be displayed in broken line with the intersecting part as a boundary as shown in FIGS. 27 and 28. It is thus possible to easily grasp in which position relation the transmission/reception plane of the real-time ultrasound image UG is placed with respect to the outline O' stored in the memory 52.

Incidentally, when FIG. 27 shows where the indicator Id corresponds to the outline Ol, FIG. 28 shows a case in which the indicator Id is displayed at the position where the outline O' is projected onto the reference medical image MG.

Advantageous effects similar to the first embodiment can be obtained even by the ultrasound diagnostic apparatus 1 of the present embodiment described above.

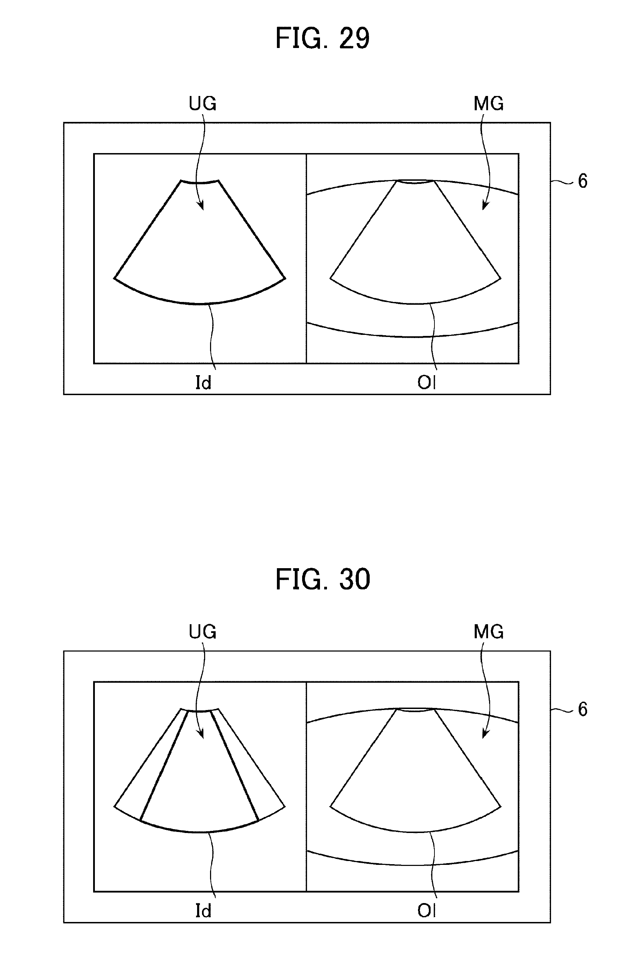

Modifications of the second embodiment will next be explained. A first modification will first be explained. In the first modification, the indicator display control unit 56 may cause the outline of a real-time ultrasound image UG to display the indicator Id as shown in FIG. 29. That is, the indicator display control unit 56 causes the outline of the ultrasound image UG to be displayed in colors corresponding to the distance between the outline O' in the volume data VD and the outline o (refer to FIG. 10) corresponding to the outline O of the transmission/reception region R of the real-time ultrasound image UG.

The indicator display control unit 56 may cause the indicator to be displayed at a position where the outline O' is projected onto the real-time ultrasound image UG as shown in FIG. 30.

A second modification will next be explained. When the parts of the ultrasound transmission/reception region R are not displayed (refer to FIG. 16), position information of an outline Og' corresponding to the outline Og (refer to FIG. 17) of the display region of the ultrasound image UG in volume data of the reference medical image is stored in the memory 52 at Step S5'. The outline Og of the display region includes the outline O of the transmission/reception region R of the ultrasound.

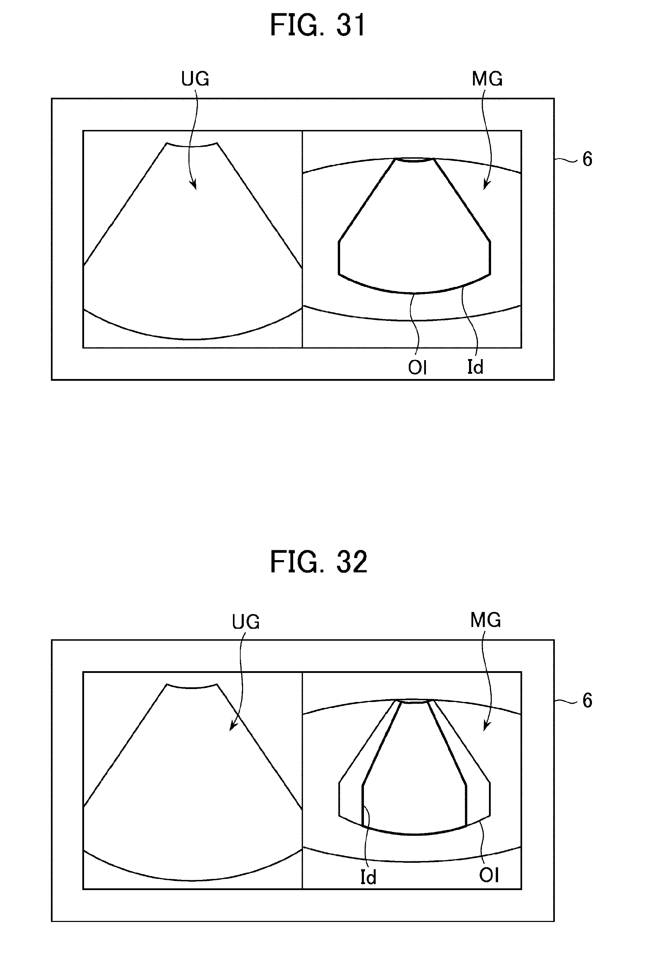

The indicator display control unit 56 causes the outline Ol (the indicator Id) in the reference medical image MG to be displayed in colors corresponding to the distance between the outline Og' in the volume data VD and the outline og (not shown) corresponding to the outline Og of the display region of the real-time ultrasound image UG as shown in FIG. 31.

The indicator display control unit 56 may cause the indicator Id to be displayed at its corresponding position where the outline Og' is projected onto the reference medical image MG as shown in FIG. 32.

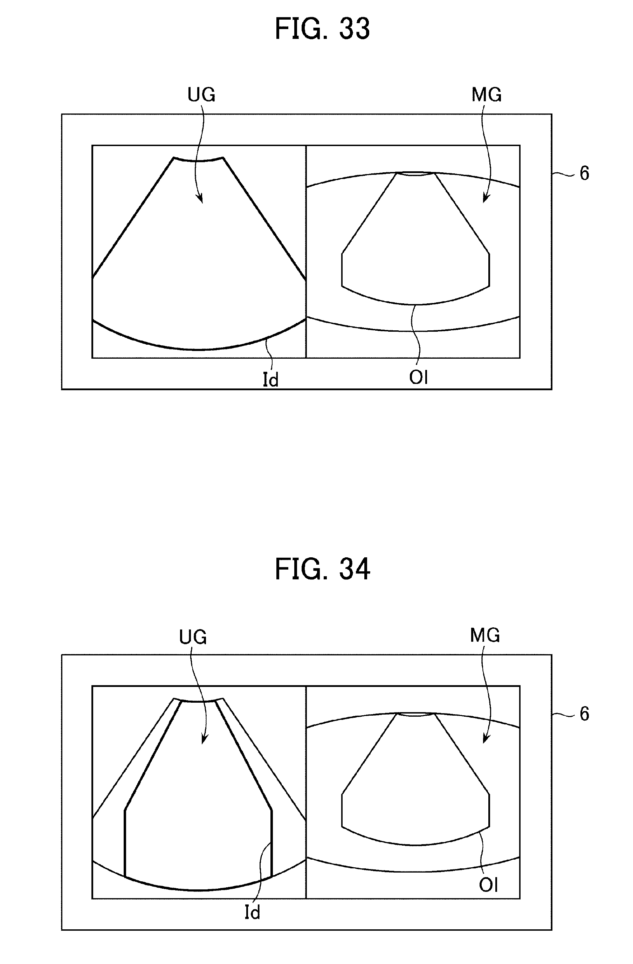

In the second modification, the indicator display control unit 56 may cause the outline of the real-time ultrasound image UG to display the indicator Id as shown in FIG. 33.

The indicator display control unit 56 may cause the indicator Id to be displayed at a position where the outline Og' is projected onto the real-time ultrasound image UG as shown in FIG. 34.

### Third embodiment

A third embodiment will next be explained. The same matters as those in the first and second embodiments will however not be described.

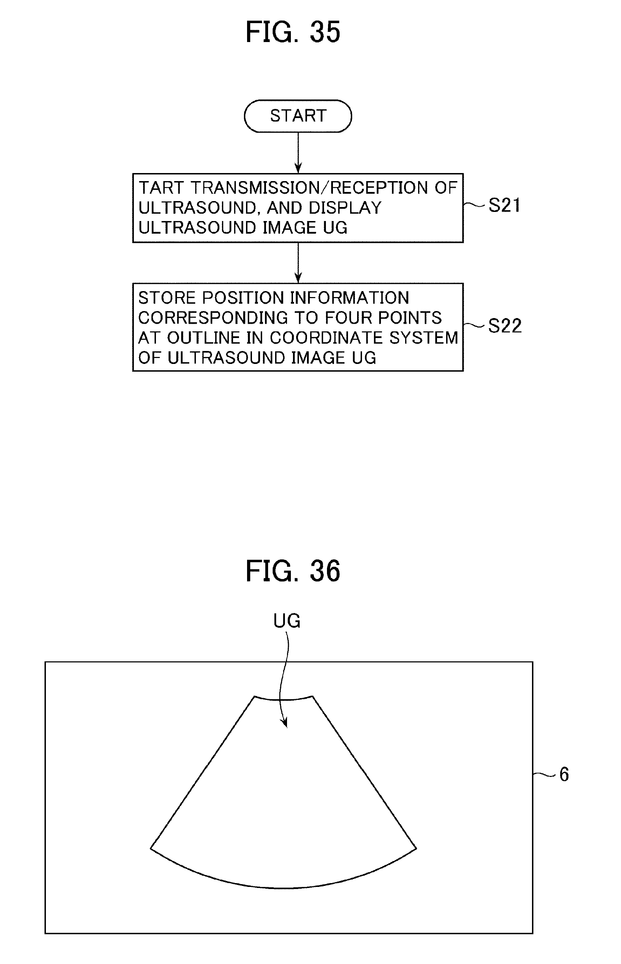

In the present embodiment, the position of insertion of a biopsy needle is determined without displaying a reference medical image. This will be explained based on the flowchart of FIG. 35. At Step S21, the operator first performs transmission/reception of ultrasound by the ultrasound probe 2 to and from a subject. The display image control unit 54 causes the display unit 6 to display an ultrasound image UG, based on thus-acquired echo signals as shown in FIG. 36.

Next, at Step S22, the operator searches for the position of insertion of a biopsy needle and its insertion angle. When the operator finds a suitable position and angle, the operator performs, through the operation unit 7, the input of storing position information of four corners C1 through C4 (refer to FIG. 6) at the outline O of an ultrasound transmission/reception region R. In the present embodiment, position information in a coordinate system of an ultrasound image UG other than volume data VD of a reference medical image is stored.

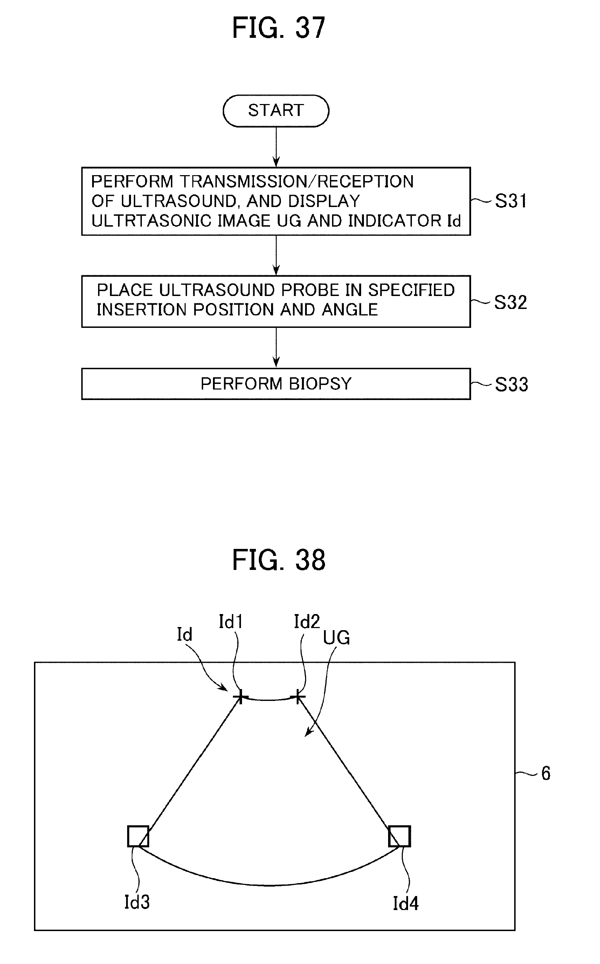

Next, the insertion of the biopsy needle in the position specified at Step S22 will be explained based on the flowchart of FIG. 37. Incidentally, in the present embodiment, the subject is assumed to be placed in the same position as when the position of insertion of the biopsy needle and its insertion angle (when the position information is stored in the memory 52) are determined at Step S22.

At Step S31, the transmission/reception of ultrasound is first performed to display an ultrasound image UG. The indicator display control unit 56 causes the indicators Id1, Id2, Id3 and Id4 to be displayed at the corners of the outline of the ultrasound image UG as shown in FIG. 38. In the present embodiment, these indicators Id1 through Id4 respectively show the distances between the four corners C1 through C4 at the outline O of the transmission/reception region R of the ultrasound stored in the memory 52 and the corners C1 through C4 at the outline O of the transmission/reception region R of the real-time ultrasound image UG.

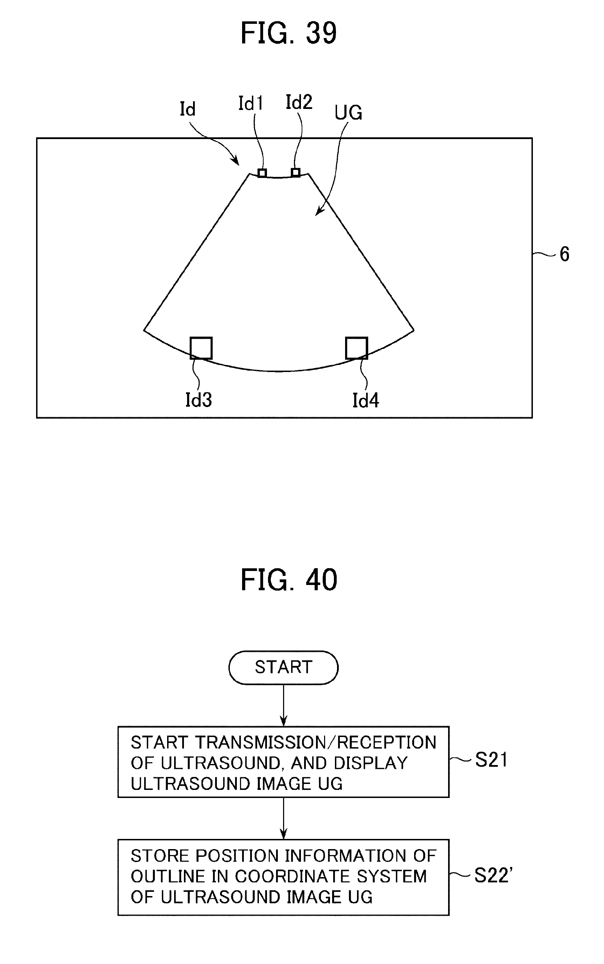

The indicator display control unit 56 may cause the indicators Id1 through Id4 to be displayed at their corresponding positions where the corners C1 through C4 at the outline O stored in the memory 52 are projected onto the real-time ultrasound image UG as shown in FIG. 39.

Next, at Step S32, the operator adjusts the position and angle of the ultrasound probe 2 in a manner similar to Step S12 in such a manner that the indicators Id1 through Id4 become graphics of "+" and thereby searches the insertion position and angle specified at Step S22. Then, the operator places the ultrasound probe 2 in the insertion position and angle both specified at Step S22.

Next, the operator inserts the biopsy needle at Step S33.

The same advantageous effects as those in the first embodiment can be obtained even by the ultrasound diagnostic apparatus 1 of the present embodiment described above.

Modifications of the third embodiments will next be explained. A first modification will first be described. As shown in the flowchart of FIG. 40, position information of an outline O of an ultrasound transmission/reception region R is stored at Step S22'. Position information in a coordinate system of an ultrasound image is stored even here.

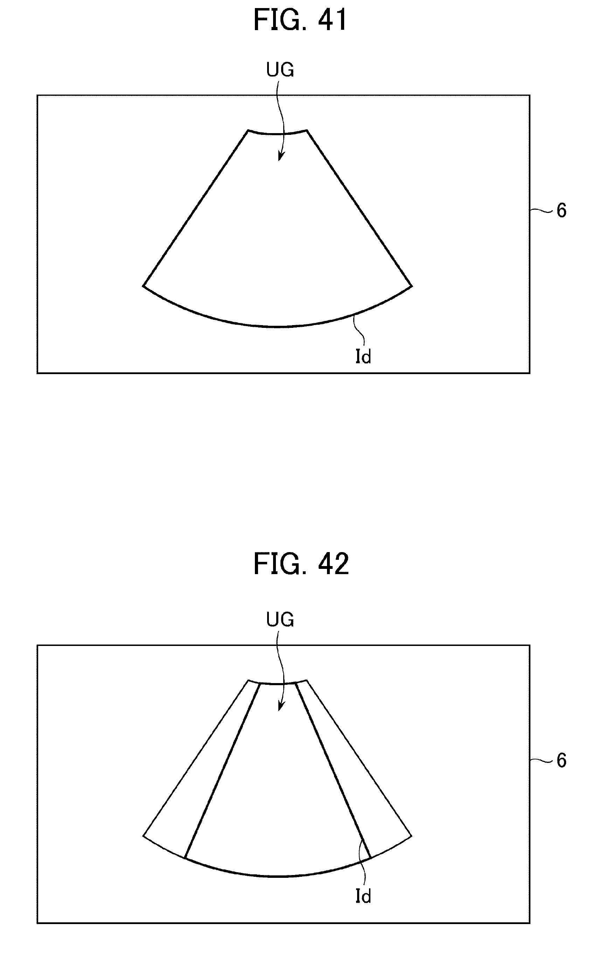

In the first modification, the indicator Id displayed at Step S31 is displayed in the outline of a real-time ultrasound image UG. Described concretely, as shown in FIG. 41, the indicator display control unit 56 causes the outline of the real-time ultrasound image UG to display an indicator Id comprised of colors each corresponding to the distance between the outline O stored at Step S22' and the outline O of the transmission/reception region R of the real-time ultrasound image UG.

The indicator display control unit 56 may cause the indicator Id to be displayed at a position where the outline O stored in the memory 52 is projected onto the ultrasound image UG as shown in FIG. 42.

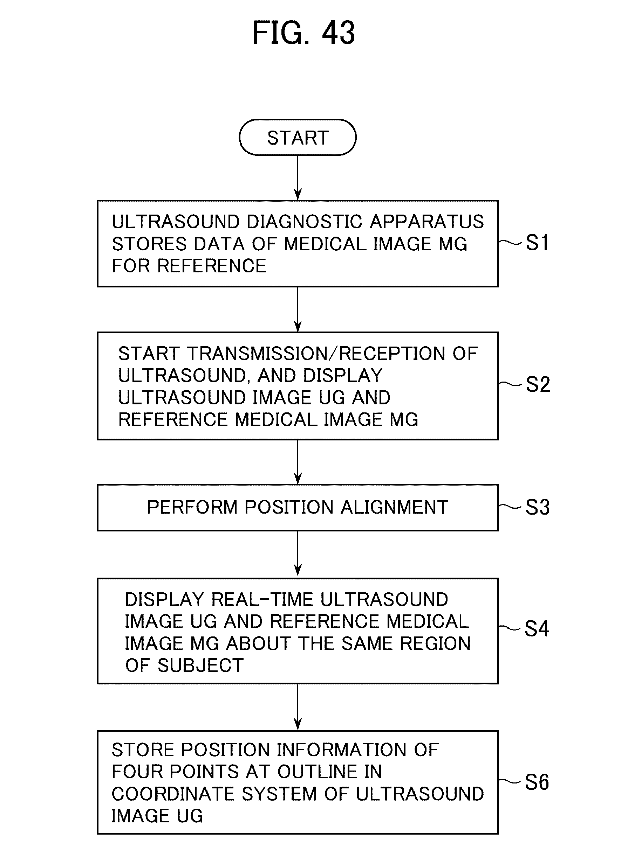

A second modification will next be explained. Even in the present modification, the reference medical image MG may be displayed. Determination of the position of insertion of the biopsy needle in the present modification will concretely be explained based on the flowchart of FIG. 43.

In FIG. 43, Steps S1 through S4 are the same as those in the flowcharts shown in FIGS. 3 and 22, and their description will therefore be omitted. At Step S6, position information about four corners C1 through C4 at an outline O of an ultrasound transmission/reception region R is stored in the memory 52. This position information is position information in a coordinate system of an ultrasound image UG.

The insertion of the biopsy needle in the position specified at Step S6 is performed in accordance with Steps S11 through S13 shown in FIG. 8. In the present modification, however, the indicator Id is displayed at the corner of the outline at the real-time ultrasound image UG in a manner similar to FIG. 14 referred to above (the indicators Id1 through Id4). The indicators Id1 through Id4 respectively show the distances between the four corners C1 through C4 at the outline O of the ultrasound transmission/reception region R stored in the memory 52, and the corners C1 through C4 at the outline O of the transmission/reception region R of the real-time ultrasound image UG.

The indicators Id1 through Id4 may respectively be displayed at positions where the four corners C1 through C4 at the outline O stored in the memory 52 are projected onto the real-time ultrasound image UG in a manner similar to FIG. 15 referred to above. The indicators Id1 through Id4 may be displayed at the corners of the outline Ol at the reference medical image MG in a manner similar to FIG. 9 referred to above. Further, the indicators Id1 through Id4 may be displayed at their corresponding positions where the corners C1 through C4 of the outline O stored in the memory 52 are projected onto the reference medical image MG, in a manner similar to FIG. 12 referred to above.

A third modification will next be explained. In the third modification, position information of the corners C1 through C4 at the outline of a display region of an ultrasound image UG is stored in the memory 52 at Step S6 shown in FIG. 43. This position information is position information in a coordinate system of the ultrasound image UG.

The indicators Id1 through Id4 are displayed on a real-time ultrasound image UG in a manner similar to FIG. 19 referred to above. The indicators Id1 through Id4 may respectively be displayed at points on the outline Ol, corresponding to the corners C1 through C4 stored in the memory 52 at a reference medical image MG in a manner similar to FIG. 18 referred to above. The indicators Id1 through Id4 may be displayed at their corresponding positions where the corners C1 through C4 stored in the memory 52 are projected onto the real-time ultrasound image UG, in a manner similar to FIG. 21 referred to above. Further, the indicators Id1 through Id4 may respectively be displayed at positions where the corners C1 through C4 stored in the memory 52 are projected onto the reference medical image MG in a manner similar to FIG. 20 referred to above.

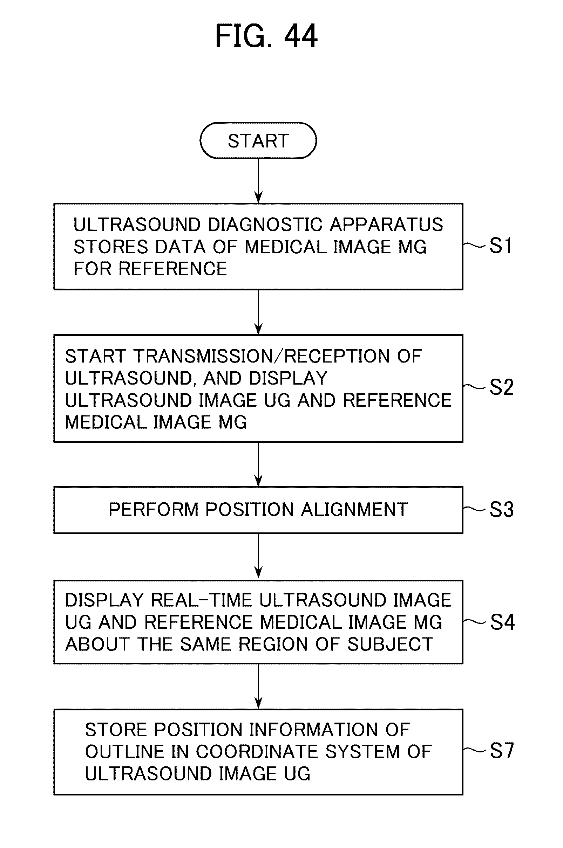

A fourth modification will next be explained. Determination of a position of insertion of a biopsy needle in the fourth modification will be explained based on the flowchart of FIG. 44. In FIG. 44, Steps S1 through S4 are identical to those in the flowcharts shown in FIGS. 3, 22 and 43. At Step S7, position information about an outline O of an ultrasound transmission/reception region R is stored in the memory 52. This position information is position information in a coordinate system of an ultrasound image UG.

The insertion of the biopsy needle in the position specified at Step S7 is performed in accordance with Steps S1 through S13 shown in FIG. 8. In the present modification, however, the indicator Id is displayed at the outline of a real-time ultrasound image UG as shown in FIG. 29 referred to above. The indicator Id may be displayed at a position where the outline O stored in the memory 52 is projected onto the real-time ultrasound image UG, as shown in FIG. 30 referred to above. The indicator Id may be the outline Ol in the reference medical image MG as shown in FIG. 24 referred to above. Further, the indicator Id may be displayed at the position where the outline O stored in the memory 52 is projected onto the reference medical image MG, as shown in FIG. 25 referred to above.

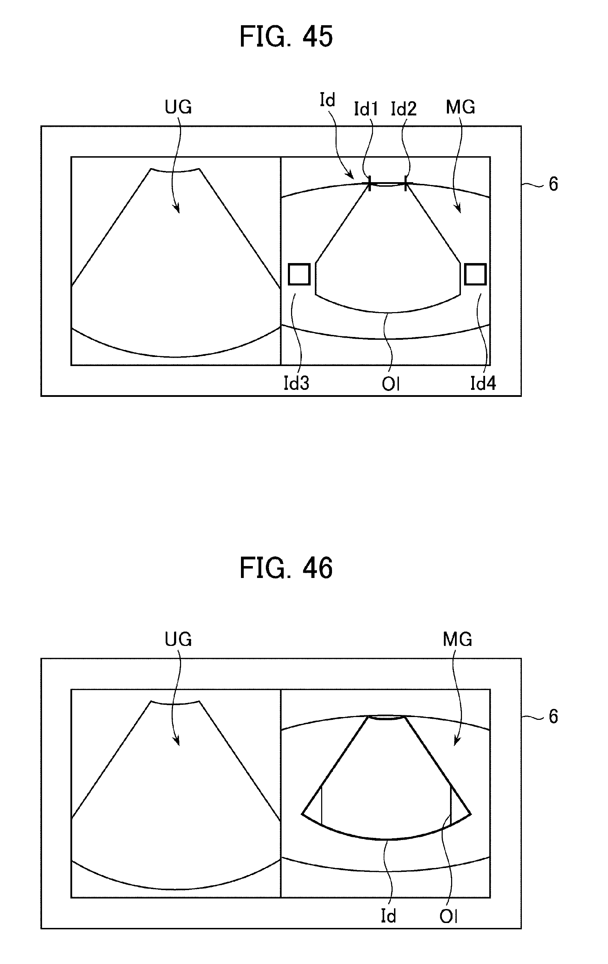

Although the invention has been explained by the embodiments as described above, it is needless to say that the invention can be changed in various ways within the scope not departing from the gist thereof. In the second modification of the first embodiment, for example, the corners C1' through C4' corresponding to the corners C1 through C4 of the transmission/reception region R may be stored in the memory 52. In this case, as shown in FIG. 45, the indicators Id1 through Id4 are respectively displayed at positions where the corners C1' through C4' are projected onto the reference medical image MG.

In the second modification of the second embodiment, the position information of the outline O' corresponding to the entirety of the outline of the transmission/reception region R in the volume data VD may be stored in the memory 52. In this case, as shown in FIG. 46, the indicator Id is displayed at its corresponding position where the outline O' stored in the memory 52 is projected onto the reference medical image MG.

Many widely different embodiments of the invention may be configured without departing from the spirit and the scope of the present invention. It should be understood that the present invention is not limited to the specific embodiments described in the specification, except as defined in the appended claims.

Various aspects and embodiments of the present invention are defined by the following numbered clauses:
1. An ultrasound diagnostic apparatus comprising:
   an ultrasound probe which performs transmission/reception of ultrasound on a subj ect; and
   an indicator display control unit which causes to be displayed indicators each indicative of a distance between at least part set in advance, of an outline of a transmission/reception region of real-time ultrasound at the subject and a corresponding position stored in advance at the outline.
2. An ultrasound diagnostic apparatus according to clause 1, wherein position information about the corresponding position stored in advance is position information in a coordinate system of volume data of a pre-acquired reference medical image of the subj ect.
3. An ultrasound diagnostic apparatus according to any preceding clause, wherein the position information is position information in a coordinate system of a three-dimensional space of the subject with a predetermined point as a point of origin.
4. An ultrasound diagnostic apparatus according to any preceding clause, wherein the coordinate system of the three-dimensional space of the subject is a coordinate system of an ultrasound image of the subject.
5. An ultrasound diagnostic apparatus according to any preceding clause, wherein the position information is position information about at least three points.
6. An ultrasound diagnostic apparatus according to any preceding clause, wherein the indicator display control unit causes the indicators to be displayed respectively at positions corresponding to at least the three points at an outline of a transmission/reception region of real-time ultrasound, which is specified at the reference medical image of the subject acquired in advance and identical in section to the real-time ultrasound image at the subject.
7. An ultrasound diagnostic apparatus according to any preceding clause, wherein the indicator display control unit causes the indicators to be displayed respectively at positions corresponding to at least the three points at the outline of the real-time ultrasound image at the subject.
8. An ultrasound diagnostic apparatus according to any preceding clause, wherein the indicator display control unit causes the indicators to be displayed respectively at positions where at least the three points are projected onto the reference medical image of the subject acquired in advance and identical in section to the real-time ultrasound image at the subject, or the real-time ultrasound image at the subject.
9. An ultrasound diagnostic apparatus according to any preceding clause, wherein the position information is position information about the entirety of the outline.
10. An ultrasound diagnostic apparatus according to any preceding clause,
   wherein the indicator display control unit causes a profile line corresponding to the outline to be displayed as the indicators on the reference medical image of the subject acquired in advance and identical in section to the real-time ultrasound image at the subject, and
   wherein the profile line has a display form corresponding to the distances.
11. An ultrasound diagnostic apparatus according to any preceding clause, wherein the indicator display control unit causes the indicators to be displayed respectively at positions where the outline stored in advance as the corresponding positions is projected, onto the reference medical image of the subject acquired in advance and identical in section to the real-time ultrasound image at the subject.
12. An ultrasound diagnostic apparatus according to any preceding clause, wherein the position information is position information corresponding to the outline of a display region of an ultrasound image at the subject.
13. An ultrasound diagnostic apparatus according to any preceding clause,
   wherein the indicator display control unit causes a profile line to be displayed, as the indicators, at a part corresponding to the outline of a display region of a real-time ultrasound image at the subject on the reference medical image of the subject acquired in advance and identical in section to the real-time ultrasound image at the subject, and
   wherein the profile line has a display form corresponding to the distances.
14. An ultrasound diagnostic apparatus according to any preceding clause, wherein the indicator display control unit causes the indicators to be displayed respectively at positions where the outline stored in advance as the corresponding positions is projected onto the reference medical image of the subject acquired in advance and identical in section to the real-time ultrasound image at the subject.
15. An ultrasound diagnostic apparatus according to any preceding clause, wherein the indicator display control unit causes indicators each having a display form corresponding to the distance to be displayed at an outline part of a real-time ultrasound image at the subj ect.
16. An ultrasound diagnostic apparatus according to any preceding clause, wherein the indicator display control unit causes the indicators to be displayed respectively at positions where the outline stored in advance as the corresponding positions is projected onto the real-time ultrasound image at the subject.
17. An ultrasound diagnostic apparatus according to any preceding clause, comprising:
   a position sensor which detects a position of the ultrasound probe;
   a position calculation unit which calculates a position of each of echo signals in a coordinate system of an ultrasound image about the subject, based on position detection information of the position sensor; and
   a display image control unit which causes a real-time ultrasound image to be displayed, specifies a region corresponding to the position calculated by the position calculation unit in volume data of the reference medical image of the subject acquired in advance and causes a reference medical image identical in section to the real-time ultrasound image to be displayed.
18. A method of ultrasound imaging comprising the steps of:
   performing transmission/reception of ultrasound on a subject; and
   displaying indicators each indicative of a distance between at least part set in advance, of an outline of a transmission/reception region of real-time ultrasound at the subject and a corresponding position stored in advance at the outline.

## Claims

1. An ultrasound diagnostic apparatus (1) comprising:
an ultrasound probe (2) which performs transmission/reception of ultrasound on a subject; and
an indicator display control unit (56) which causes to be displayed indicators each indicative of a distance between at least part set in advance, of an outline of a transmission/reception region of real-time ultrasound at the subject and a corresponding position stored in advance at the outline.

2. An ultrasound diagnostic apparatus (1) according to claim 1, wherein position information about the corresponding position stored in advance is position information in a coordinate system of volume data of a pre-acquired reference medical image of the subj ect.

3. An ultrasound diagnostic apparatus (1) according to any preceding claim, wherein the position information is position information in a coordinate system of a three-dimensional space of the subject with a predetermined point as a point of origin.

4. An ultrasound diagnostic apparatus (1) according to any preceding claim, wherein the coordinate system of the three-dimensional space of the subject is a coordinate system of an ultrasound image of the subject.

5. An ultrasound diagnostic apparatus (1) according to any preceding claim, wherein the position information is position information about at least three points.

6. An ultrasound diagnostic apparatus (1) according to any preceding claim, wherein the indicator display control unit (56) causes the indicators to be displayed respectively at positions corresponding to at least the three points at an outline of a transmission/reception region of real-time ultrasound, which is specified at the reference medical image of the subject acquired in advance and identical in section to the real-time ultrasound image at the subject.

7. An ultrasound diagnostic apparatus (1) according to any preceding claim, wherein the indicator display control unit (56) causes the indicators to be displayed respectively at positions corresponding to at least the three points at the outline of the real-time ultrasound image at the subject.

8. An ultrasound diagnostic apparatus (1) according to any preceding claim, wherein the indicator display control unit (56) causes the indicators to be displayed respectively at positions where at least the three points are projected onto the reference medical image of the subject acquired in advance and identical in section to the real-time ultrasound image at the subject, or the real-time ultrasound image at the subject.

9. An ultrasound diagnostic apparatus (1) according to any preceding claim, wherein the position information is position information about the entirety of the outline.

10. An ultrasound diagnostic apparatus (1) according to any preceding claim,
wherein the indicator display control unit (56) causes a profile line corresponding to the outline to be displayed as the indicators on the reference medical image of the subject acquired in advance and identical in section to the real-time ultrasound image at the subj ect, and
wherein the profile line has a display form corresponding to the distances.

11. An ultrasound diagnostic apparatus (1) according to any preceding claim, wherein the indicator display control unit (56) causes the indicators to be displayed respectively at positions where the outline stored in advance as the corresponding positions is projected, onto the reference medical image of the subject acquired in advance and identical in section to the real-time ultrasound image at the subject.

12. An ultrasound diagnostic apparatus (1) according to any preceding claim, wherein the position information is position information corresponding to the outline of a display region of an ultrasound image at the subject.

13. An ultrasound diagnostic apparatus (1) according to any preceding claim, wherein the indicator display control unit (56) causes indicators each having a display form corresponding to the distance to be displayed at an outline part of a real-time ultrasound image at the subject.

14. An ultrasound diagnostic apparatus (1) according to any preceding claim, comprising:
a position sensor (10) which detects a position of the ultrasound probe (2);
a position calculation unit (51) which calculates a position of each of echo signals in a coordinate system of an ultrasound image about the subject, based on position detection information of the position sensor (10); and
a display image control unit (5) which causes a real-time ultrasound image to be displayed, specifies a region corresponding to the position calculated by the position calculation unit (51) in volume data of the reference medical image of the subject acquired in advance and causes a reference medical image identical in section to the real-time ultrasound image to be displayed.

15. A method of ultrasound imaging comprising the steps of:
performing transmission/reception of ultrasound on a subject; and
displaying indicators each indicative of a distance between at least part set in advance, of an outline of a transmission/reception region of real-time ultrasound at the subject and a corresponding position stored in advance at the outline.
